# EUROPEAN PATENT SPECIFICATION

(11) **EP 4 101 400 B1**
(45) Date of publication and mention of the grant of the patent: **30.10.2024**
(21) Application number: 21924731.9
(22) Date of filing: 11.06.2021
(51) Int. Cl.: A61B 17/29

(54) **SURGICAL INSTRUMENT**
CHIRURGISCHES INSTRUMENT
INSTRUMENT CHIRURGICAL

(30) Priority: 02.02.2021 JP 2021014793
(43) Date of publication of application: 14.12.2022
(73) Proprietor: Medmetalex Co., Ltd, Nishiyodogawa-ku Osaka-shi, Osaka 555-0012 (JP)
(72) Inventor: AMINO, Hirokazu, Osaka-shi, Osaka 555-0012 (JP)
(74) Representative: VKK Patentanwälte PartG mbB
(86) International application number: PCT/JP2021/022244
(87) International publication number: WO 2022/168342

(56) References cited:
- JP-A- 2018 094 290
- JP-A- 2018 504 992
- US-A1- 2016 324 514
- US-A1- 2016 324 514
- US-A1- 2018 256 182

## Description

### [Technical Field]

The present invention relates to a surgical instrument used for minimally invasive surgery such as laparoscopic surgery.

### [Background Art]

Surgical instruments used for minimally invasive surgery such as laparoscopic surgery require a high degree of freedom in the head section attached to the distal end of the surgical instrument. Since it is necessary for a surgeon operating a surgery of a patient (surgical subject) to grip the surgical instrument that is partially inserted into the body while manipulating the head portion, such a surgical instrument must be the one capable of being easily manipulated by the surgeon's hand while achieving fine head movements.

To achieve fine head movements, the surgical instrument is required to reliably transmit, by a simple hand manipulation, the movement from the manipulating side (proximal side near the surgeon) to the side where the head portion is positioned (distal side apart from the surgeon).

The invention described in Patent Literature 1 relates to a surgical instrument used in laparoscopic surgery or the like. The surgical instrument is roughly configured of, in the order from distal end side; a tool head, a central system, a main body portion, and a proximal handle. US2018/256182 A1 and US2016/324514 A1 disclose similar devices of the prior art. [Other Prior Art Documents]

### [Patent Documents]

[PTL 1] WO 2016/162883

### [Summary of the Invention]

Claim 1 defines the invention and dependent claims disclose embodiments. No surgical methods are claimed.

### [Problems to be Solved by the Invention]

With the surgical instrument described in PTL 1, the tool head at the distal end can move freely in lateral direction by a hand manipulation of a surgeon, and the body tissue can be dissected if, for example, the tool head has a function of scissors.

In the surgical instrument described in PTL 1, the function to convert the surgeon's hand manipulation into a movement of a tool head primarily relies on the central system 3. To assemble the central system 3, numerous components are required, including a third outer shaft 3a, a second intermediate shaft 3b, a center rod 3c, a universal joint 3d, a rigid slider 3e, a cylinder 3f, a rear rotating collar 3g, rigid links 3h and 3i, a front cover 2a, a rigid tubular rod 2b, a front rotating collar 2c, and so on.

Since a surgical instrument is used in an area very close to the surgical field inside the patient's body, and is used for surgery that must be performed within a limited time, such a surgical instrument should never cause any failure during surgery and thus is required to be robust. If any component of the surgical instrument falls into the surgical field, the component must be positioned and removed, which can be a major problem. There is thus contradictory between the ability to freely move the tool head at the distal end and the safety assurance required for such a surgical instrument.

The present invention is made in consideration of the above respect, and the object is to provide a surgical instrument used for minimally invasive surgery such as laparoscopic surgery, in which the head can be freely moved by a simple hand manipulation by a surgeon, and in which the number of components is reduced to ensure high safety.

### [Means to Solve the Problem]

Thepresent disclosure relating to the present invention made to solve the above-mentioned problem, is a surgical instrument comprising: at a distal portion, a tool head having longitudinal and lateral degrees of freedom; and at a proximal portion, a first manipulator to control the longitudinal degrees of freedom and a second manipulator to control the lateral degrees of freedom; wherein: the tool head includes a movable portion having longitudinal degrees of freedom and a base portion that defines a lateral orientation of the tool head; the movable portion and the first manipulator are connected by a first connecting structure; the base portion and the second manipulator are connected by a second connecting structure; the first connecting structure includes a first conversion mechanism that converts linear motion from the first manipulator to rotational motion, a first shaft that is to be rotated by the first conversion mechanism, a universal joint provided at a distal end portion of the first shaft, a second conversion mechanism connected to the universal joint to convert rotational motion into linear motion, and a crank provided at a distal end portion of the second connecting structure and connected to a proximal end portion of the movable portion; the second conversion mechanism transmits rotational motion of the second manipulator, provided with a tubular shaft in which the first shaft is coaxially encompassed and a pinion is formed at the distal portion, and a rack base portion being linked to the base portion where a rack to be meshed with the pinion being formed thereon in an approximate semi-circular shape; and an intersection of shaft centers of two pivot shafts of the universal joint is positioned on an axial center of rotational axis of the rack.

The surgical instrument has two manipulators, the first manipulator and the second manipulator, which are the manipulators to be manipulated by a surgeon. The first manipulator is for moving the movable portion in a longitudinal direction, and the second manipulator is for moving the base portion in a lateral direction.

There provided the first connecting structure for connecting the first manipulator and the tool head and the second connecting structure for connecting the second manipulator and the tool head. The first connecting structure includes: the first conversion mechanism that converts linear motion from the first manipulator to rotational motion; the first shaft that is to be rotated by the first conversion mechanism, the universal joint provided at the distal end portion of the first shaft; the second conversion mechanism connected to the universal joint to convert rotational motion into linear motion; and the crank provided at the distal end portion of the second conversion mechanism and connected to the proximal end portion of the movable portion. This first connecting structure transmits the manipulation from the first manipulator to the tool head.

The second connecting structure is configured to transmit rotational motion from the second manipulator, the second connecting structure including: a tubular shaft coaxially encompassing the first shaft and having a pinion formed at the distal portion, and a rack base portion where a rack to be meshed with the pinion is formed thereon in an approximate semi-circular shape. This second connecting structure transmits the manipulation from the second manipulator to the tool head.

Since the intersection of shaft centers of two pivot shafts of the universal joint is positioned on an axial center of rotational axis of the rack, even when the orientation of the base portion linked to the rack base portion is changed laterally to a certain angle by the manipulation from the second manipulator, the movable portion also moves laterally in the same angle, and further, the movable potion can be oriented in a longitudinal direction by the manipulation from the first manipulator. With this structure, it is possible to provide a surgical instrument in which the tool head can be freely moved by a simple hand manipulation, and in which the number of components is reduced to ensure high safety.

The surgical instrument of the present disclosure is characterized in that: the first conversion mechanism includes a first tubular body in which the first shaft penetrates therethrough; a first helical groove is formed on a side wall of the first tubular body; a first slide element that is to be guided by the first helical groove to slide is formed on the first shaft; on the outer surface of the first tubular body, a first tubular body engaging portion that engages with the first manipulator to transmit linear motion from the first manipulator to the first tubular body is formed; and when the first manipulator is moved, the first tubular body moves linearly, and the first slide element is guided by the first helical groove, which allows the first shaft to rotate.

Since the linear motion of the first manipulator is converted to the rotary motion of the first shaft by a combination of the first tubular body on which the first helical groove is formed and the first shaft having the first slide element, the first shaft can be rotated by the motion from the first manipulator with the simple structure.

This surgical instrument of the present disclosure is characterized in that: the second conversion mechanism includes a second tubular body that is rotated in connection with the universal joint; on the side surface of the second tubular body, a second helical groove is formed and a second shaft connected to the proximal end of the crank is provided; a second slide element to be guided to slide by the second helical groove is formed on the second shaft; and when the second tubular body is rotated, the second slide element is guided by the second helical groove, which allows the second shaft to perform liner motion.

Since the combination of the second tubular body on which the second helical groove is formed and the second shaft having the second slide element enables to convert the rotary motion of the first shaft into the linear motion of the second shaft, with the simple structure, the second shaft can be linearly moved by the movement of the first manipulator, the movement of which is transmitted to the crankshaft so as to allow the movable portion to move.

The surgical instrument according to any of the present disclosure, comprising a function of forceps to grip a living tissue, by means of the movable portion and the base portion of the tool head.

The surgical instrument according to the present disclosure is applicable to surgical instruments imparted with various functions. The present disclosure is one of such instruments. The tool head is imparted with the function of a so-called bioptome. By simply forming the shape of the movable portion and the shape of the base portion of the tool head so as to be suitable for gripping a part of the body tissue, it is possible to impart a safe and flexible bioptome function.

The surgical instrument according to any of the present disclosure, provided with a function of scissors for cutting living tissue, by means of the movable portion and the base portion of the tool head.

The disclosure is a surgical instrument in which the tool head is imparted with a so-called a function of scissors. By simply forming the shape of the movable portion and the shape of the base portion of the tool head so as to be suitable for cutting parts of the body tissue, it is possible to impart a safe and flexible function of scissors.

The surgical instrument according to any of the disclosure, provided with an endoscopic function, wherein a camera is mounted on the movable portion of the tool head for internally inspecting the body.

With a camera being mounted at the movable portion of the tool head, the surgical instrument of the present disclosure can play a role of an endoscope that can be freely oriented in both lateral and longitudinal directions.

### [Effect of the invention]

According to the present invention, it is possible to provide a surgical instrument used for minimally invasive surgery such as laparoscopic surgery, in which the head can be freely moved by a simple hand manipulation by a surgeon, and in which the number of components is reduced to ensure high safety.

### [Brief description of the drawings]

Fig. 1 is a perspective diagram illustrating the entirety of a surgical instrument having a function of bioptome according to Embodiment 1 of the present invention.
Fig. 2 is a perspective diagram illustrating a part inside the surgical instrument of Embodiment 1.
Fig. 3 is a perspective diagram illustrating an enlarged view of the area near the first conversion mechanism portion the surgical instrument of Embodiment 1.
Fig. 4 is a perspective diagram illustrating the second conversion mechanism portion and its peripheral portion of the surgical instrument of Embodiment 1.
Fig. 5 is a perspective diagram illustrating the second conversion mechanism portion and its peripheral portion of the surgical instrument of Embodiment 1, in a state where an outer tube is mounted.
Fig. 6 is a perspective diagram illustrating the second conversion mechanism portion and its peripheral portion of the surgical instrument of Embodiment 1, in a state when the second shaft is placed to the left side.
Fig. 7 is a schematic diagram illustrating the second conversion mechanism portion and its peripheral portion, and the movement of the movable portion.
Fig. 8 is a perspective diagram illustrating the second connecting structure and its peripheral portion of the surgical instrument of Embodiment 1.
Fig. 9 is an enlarged perspective diagram illustrating the pinion and rack portion of the second connecting structure and its peripheral portion of the surgical instrument of Embodiment 1.
Fig. 10 is a perspective diagram illustrating the tool head portion and its peripheral portion of the surgical instrument of Embodiment 1 of the present invention, provided with a function of bioptome.

### [Description of Embodiments]

Embodiments of the present disclosure will be hereinafter described by referring to the drawings. It should be noted that the following embodiments are illustrative examples that are preferred in nature and are not intended to limit the scope of the invention, its applications, or uses.

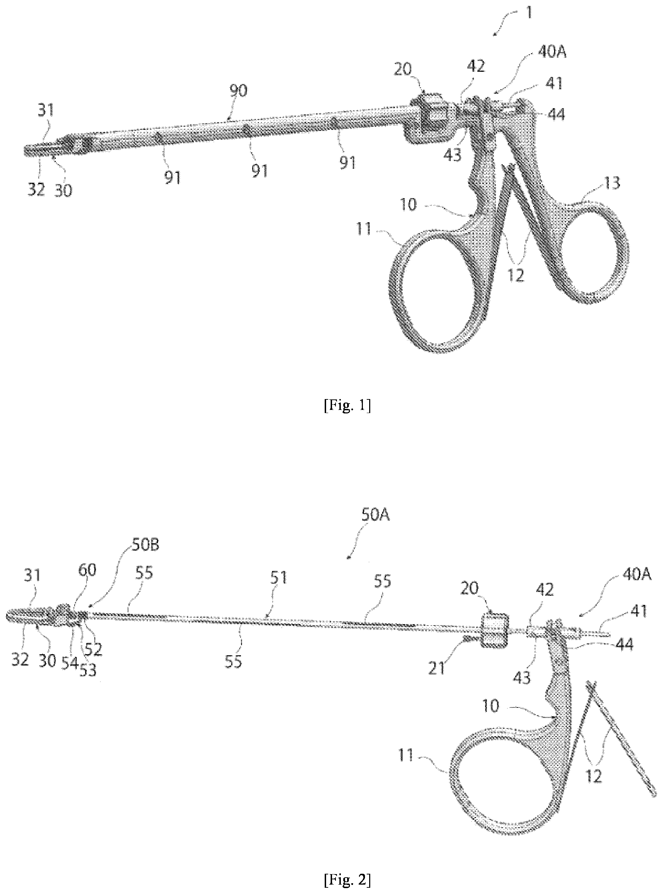

Fig. 1 is a perspective diagram of the surgical instrument 1 according to Embodiment 1 of the present invention provided with a function of bioptome, there illustrated a first manipulator 10 and a second manipulator 20, a tool head 30 and a cover 90 that encompasses a member for linking both manipulators 10 and 20 to the tool head 30. Holes 91 drilled in the cover 90 are provided for facilitating post-operative cleaning.

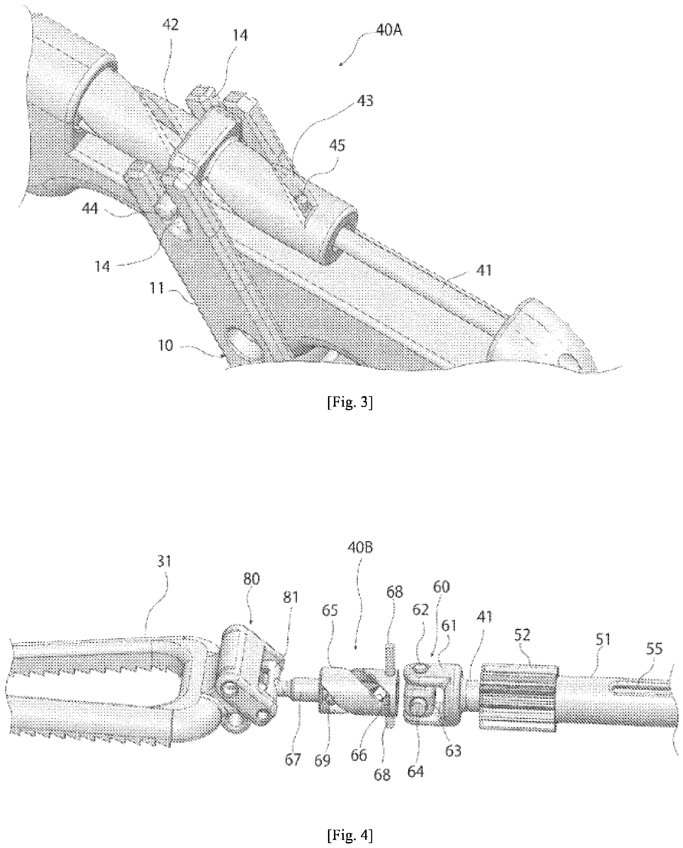

The first manipulator 10 is formed in a handle type, composed of a manipulation movable portion 11, a plate spring 12, and a manipulation fixing portion 13, but the form of the first manipulator 10 is not limited to this type. A first conversion mechanism 40A is shown above the first manipulator 10. The first conversion mechanism 40A is configured such that a first shaft 41 is rotated by the movement of a first tubular body 42, which is linked to the movement of the first manipulator 10 via a first tubular body engaging portion 44. A first helical groove 43 plays a role to convert this linear motion into rotary motion, which mechanism will be explained in detail in the description of Fig 3. The tool head 30 is provided with a base portion 32 of the tool head 30 to be oriented in the lateral direction with respect to the axial direction of the cover 90, and a movable portion 31 of the tool head 30 to be oriented in the longitudinal direction with respect to the axial direction of the cover 90.

Fig. 2 shows the surgical instrument 1 of Fig. 1 in a state where the cover 90 is removed. A first connected structure 50A illustrated in Fig. 2 is a connected structure that connects the first manipulator 10 and the movable portion 31 of the tool head 30. A second connected structure 50B is a connected structure that connects the second manipulator 20 and the base portion 32 of the tool head 30. A tubular shaft 51 is configured to transmit the rotation of the second manipulator 20 directly as rotary motion to a pinion 52 at the distal end. The pinion 52 meshes with a rack 54 arranged on a rack base portion 53 so as to allow the rack base portion 53 to rotate. The rack base portion 53 is directly connected to the base portion 32 of the tool head 30, thereby the base portion 32 is commensurately rotated. The tubular shaft 51 is provided with multiple longitudinal grooves 55, which are designed to facilitate the cleaning of the instrument after surgery. A second manipulator-rotation stopper 21 shown in the drawing is a member for preventing the second manipulator 20 from rotating inadvertently, after the completion of intended manipulation performed by rotating the second manipulator 20.

Fig. 3 is a perspective diagram illustrating the enlarged view of a portion of the first conversion mechanism 40A of the medical instrument of Embodiment 1. An engaging recess 14 formed in the upper part of the manipulation movable portion 11 and a first tubular body engaging portion 44 attached on the outer surface of the first tubular body 42 are engaged with each other, thereby the movement of the first manipulator 10 is transmitted to the first tubular body 42, which allows the first tubular body 42 to move forward and backward. Along with the above-mentioned movements, a first slide element 45 formed on the first shaft 41 is moved along the first helical groove 43, which allows the first shaft 41 to rotate.

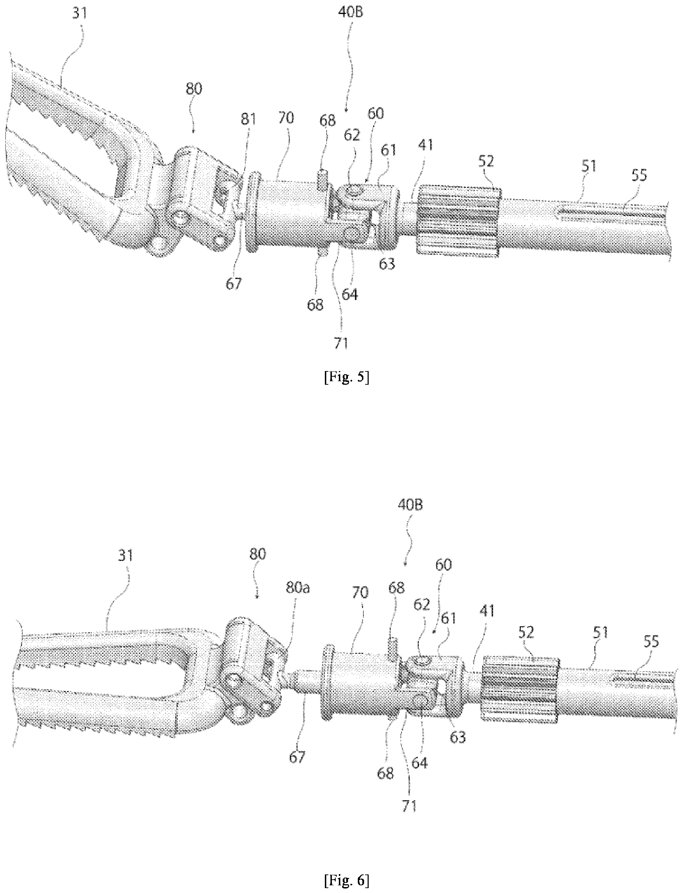

The movement of the second conversion mechanism 40B will be explained with reference to Figs. 4-6. A universal joint 60 is attached at the distal end of the first shaft 41. The universal joint 60 is provided with a first bifurcated piece 61, an intermediate body 63, a first pivot shaft 62 and a second pivot shaft 64. A second tubular body 65 at the distal end side is encompassed in an outer tube 70 (see Fig. 5), and the outer tube 70 is provided with a second bifurcated piece 71 (see Fig. 5) to be linked to the second pivot shaft 64. Protrusions 68 are for connecting the second tubular body 65 and the outer tube 70, but since Fig 4. illustrates the state where the outer tube 70 is removed, the first shaft 41 and the tool head 30 are not yet connected. There are two protrusions 68 on top and bottom, but the protrusions may be formed of a single bar body.

A second helical groove 66 is cut in the second tubular body 65, and a second slide element 69 attached to the second shaft 67 that is connected to a proximal end 80a of a crank 80 is guided along the second helical groove 66 to move forward and backward in association with the rotation of the second tubular body 65. The forward and backward movements are transmitted to the crank 80, which cause a change in longitudinal orientation of the movable portion 31 of the crank 30.

Fig. 5 illustrates a state where the outer tube 70 is mounted, with the second bifurcated piece 71 being connected to the second pivot shaft 64. Fig. 5 shows a state where the movable portion 31 is opened, in a state where the second slide element 69 is moved to the most proximal side (right side in the drawing). In contrast, Fig. 6 shows a state where the movable portion 31 is closed, in a state where the second slide element 69 is moved to the most distal side (left side in the drawing). This state is the same as shown in Fig. 4.

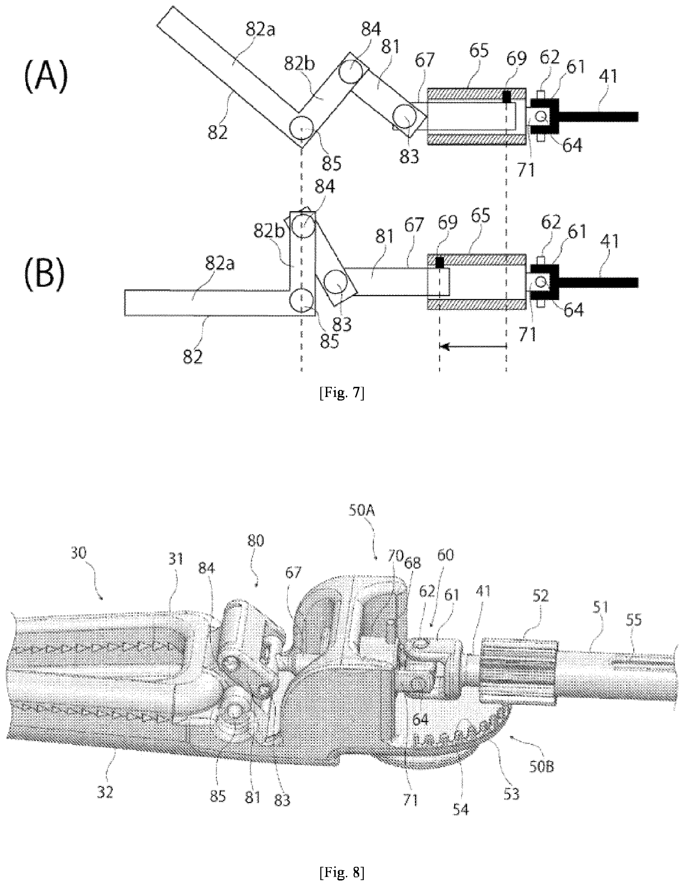

Fig. 7 schematically shows the movement of the crank 80. Fig. 7 (A) shows a state where the second slide element 69 is moved to the most proximal side. An L-shaped body 82 is composed of an L-shaped body first portion 82a and an L-shaped body second portion 82b, and the L-shaped body first portion 82a corresponds to the movable portion 31 of the tool head 30. The second shaft 67 is connected to the proximal end portion of the L-shaped second portion 82b by an arm 81, and the second shaft and arm 81 are connected pivotably about a first axis 83. The arm 81 and the L-shaped second portion 82b are connected pivotably about a second axis 84. A third shaft 85 is provided for allowing the L-shaped body 82 to pivot thereabout as a pivot shaft.

Fig. 7 (B) shows that the second slide element 69 is moved from the position shown in Fig. 7A to a position at the most distal end side by the rotation of the first shaft 41. When comparing Fig. 7(A) and Fig. 7(B), the second slide element 69 moves from the dotted position P₁ to the dotted position P₂ by the distance indicated with an arrow, and by the same distance of which, the second shaft 67 axially displaced. The second shaft 67 is moved to the distal side and the arm 81 is vertically oriented, and the L-shaped body 82 (the movable portion 31 of the tool head 30) is displaced so as to allow the L-shaped body first portion 82a to be horizontal oriented. A position P₃ of the third axis 85 is configured to be fixed.

Fig. 8 is a diagram illustrating the movement of the present surgical instrument 1 in a state where the first conversion structure 50A and the second conversion structure 50B are combined. When the second manipulator 20 is rotated by hand, the pinion 52 mounted on the distal end of the tubular shaft 51 is rotated while being meshed with the rack 54 formed in an approximate semi-circular shape on the rack base portion 53 so that the rack base portion 53 is rotated, and since the rack base portion 53 and the base portion 32 of the tool head 30 are connected, the orientation of the base portion 32 is changed.

This manipulation causes the universal joint 60 to change the orientation of the movable portion 31 about the first pivot shaft 62 by the same angle as that of the base portion 32. The above is enabled by the configuration such that the intersection of the shaft centers of the first pivot shaft 62 and the second pivot shaft 64 are positioned on the axial center of the rotational axis of the rack base portion 53. With the above configuration, in commensuration with the movement of the base portion 32, which is rotated by the same angle as the rotation of the rack base portion 53, the movable portion 31 is rotated by the same angle, thereby the first conversion mechanism 40A allows the movable portion 31 to move longitudinally.

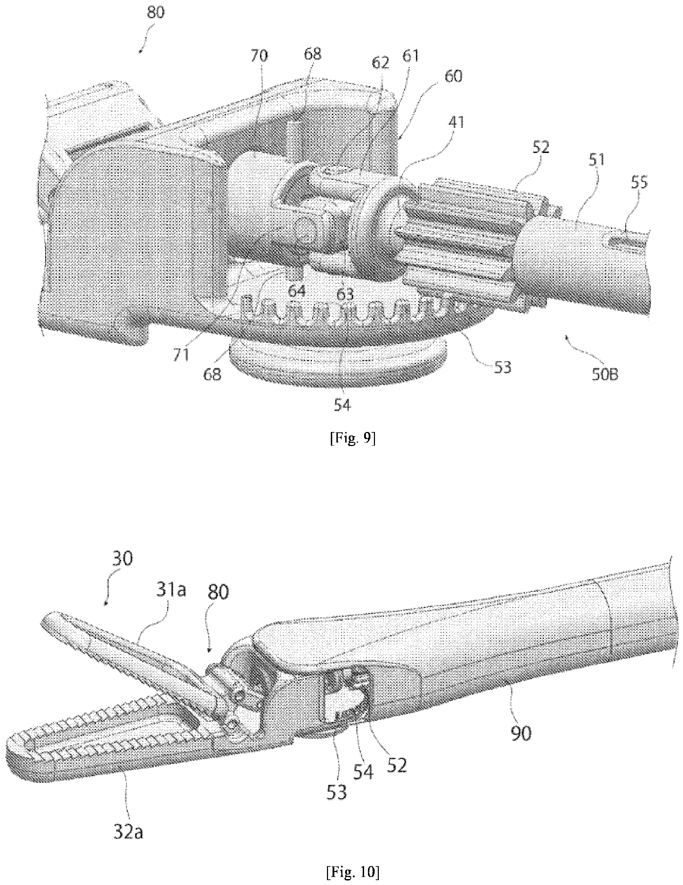

Fig. 9 is a perspective diagram illustrating the second connecting structure 50B when viewed from the proximal side.

Fig. 10 is a perspective diagram illustrating the distal end portion of the surgical instrument 1 provided with a function of forceps to grip the biological tissue. The movable portion 31 is an upper gripping portion 31a and the base portion 32 is a lower gripping portion 32a. By variously modifying the form of the distal end portion as mentioned above, it is possible to provide a surgical instrument with a function of scissors, or a surgical instrument with a function of endoscope, equipped with a camera for internally inspecting the body.

### [Industrial Applicability]

As explained above, the surgical instrument of the present invention is the one used for minimally invasive surgery such as laparoscopic surgery, in which the head can be freely moved by a simple hand manipulation by a surgeon, and in which the number of components is reduced to ensure high safety. The scope of the invention is defined by the claims.

### [Description of Reference Numerals]

1: surgical instrument
10: first manipulator
11: manipulation movable portion
12: plate spring
13: manipulation fixing portion
14: engaging recess
20: second manipulator
21: second manipulator-rotation stopper
30: tool head
31: movable portion
31a: upper gripping portion
32: base portion
32a: lower gripping portion
40A: first conversion mechanism
40B: second conversion mechanism
41: first shaft
42: first tubular body
43: first helical groove
44: first tubular body engaging portion
45: first slide element
50A: first conversion structure
50B: second conversion structure
51: tubular shaft
52: pinion
53: rack base portion
54: rack
55: longitudinal groove
60: universal joint
61: first bifurcated piece
62: first pivot shaft
63: intermediate body
64: second pivot shaft
65: second tubular body
66: second helical groove
67: second shaft
68: protrusion
69: second slide element
70: outer tube
71: second bifurcated piece
80: crank
80a: proximal end
81: arm
82: L-shaped body
82a: L-shaped body first portion
82b: L-shaped body second portion
83: first axis
84: second axis
85: third axis
90: cover
91: hole

## Claims

1. A surgical instrument comprising:
at a distal portion, a tool head (30) having longitudinal and lateral degrees of freedom; and at a proximal portion, a first manipulator (10) to control the longitudinal degrees of freedom and a second manipulator (20) to control the lateral degrees of freedom; wherein
the tool head includes a movable portion (31) having longitudinal degrees of freedom and a base portion (32) that defines a lateral orientation of the tool head,
the movable portion and the first manipulator are connected by a first connecting structure,
the base portion and the second manipulator are connected by a second connecting structure,
the first connecting structure includes: a first conversion mechanism (40A) that converts linear motion from the first manipulator to rotational motion; a first shaft (41) that is to be rotated by the first conversion mechanism; a universal joint (60) provided at a distal end portion of the first shaft; a second conversion mechanism (40B) that is connected to the universal joint to convert rotational motion into linear motion; and a crank (80) provided at a distal end portion of the second conversion mechanism and connected to a proximal end portion of the movable portion;
the second connecting structure is configured to transmit rotational motion of the second manipulator, and provided with: a tubular shaft (51) in which the first shaft is coaxially encompassed and a pinion (52) is formed at a distal portion; and a rack base portion (53) being linked to the base portion, on which a rack to be meshed with the pinion is formed in an approximate semi-circular shape;
and an intersection of shaft centers of two pivot shafts (62,64) of the universal joint is positioned on an axial center of rotational axis of the rack.

2. The surgical instrument according to claim 1, wherein: the first conversion mechanism includes a first tubular body (42) through which the first shaft penetrates; a first helical groove (43) is formed on a side wall of the first tubular body; a first slide element (45) that is guided by the first helical groove to slide is formed on the first shaft; a first tubular body engaging portion (44) that engages with the first manipulator and transmits linear motion from the first manipulator to the first tubular body is formed on the outer surface of the first tubular body; and when the first manipulator is moved, the first tubular body is moved linearly, and the first slide element is guided by the first helical groove to allow the first shaft to rotate.

3. The surgical instrument according to claim 1 or claim 2, wherein: the second conversion mechanism includes a second tubular body (65) that is rotated in connection with the universal joint; a second helical groove (66) is formed on the side surface of the second tubular body; a second shaft (67) connected to the proximal end of the crank is provided; a second slide element (69) to be guided by the second helical groove to slide is formed on the second shaft; and when the second tubular body is rotated, the second slide element is guided by the second helical groove to allow the second shaft to perform a liner motion.

4. The surgical instrument according to any one of claims 1 to 3, provided with a function of forceps to grip a living tissue, by means of the movable portion and the base portion of the tool head.

5. The surgical instrument according to any one of claims 1 to 3, provided with a function of scissors for cutting living tissue, by means of the movable portion and the base portion of the tool head.

6. The surgical instrument according to any one of claims 1 to 3, provided with an endoscopic function, with a camera being mounted on the movable portion of the tool head for internally inspecting the body.

## Patentansprüche

1. Ein chirurgisches Instrument, das Folgendes umfasst:
an einem distalen Abschnitt einen Werkzeugkopf mit Längs- und Querfreiheitsgraden; und an einem proximalen Abschnitt einen ersten Manipulator zur Steuerung der Längsfreiheitsgrade und einen zweiten Manipulator zur Steuerung der Querfreiheitsgrade; wobei
der Werkzeugkopf einen beweglichen Teil mit Freiheitsgraden in Längsrichtung und ein Basisteil umfasst, der eine seitliche Ausrichtung des Werkzeugkopfs definiert,
der bewegliche Teil und der erste Manipulator durch eine erste Verbindungsstruktur verbunden sind,
das Basisteil und der zweite Manipulator durch eine zweite Verbindungsstruktur verbunden sind,
die erste Verbindungsstruktur umfasst: einen ersten Umwandlungsmechanismus, der eine lineare Bewegung des ersten Manipulators in eine Drehbewegung umwandelt; eine erste Welle, die durch den ersten Umwandlungsmechanismus gedreht werden soll; ein Universalgelenk, das an einem distalen Endabschnitt der ersten Welle vorgesehen ist; einen zweiten Umwandlungsmechanismus, der mit dem Universalgelenk verbunden ist, um eine Drehbewegung in eine lineare Bewegung umzuwandeln; und eine Kurbel, die an einem distalen Endabschnitt des zweiten Umwandlungsmechanismus vorgesehen und mit einem proximalen Endabschnitt des beweglichen Abschnitts verbunden ist;
die zweite Verbindungsstruktur so konfiguriert ist, dass sie eine Drehbewegung des zweiten Manipulators überträgt, und versehen ist mit: einer rohrförmigen Welle, in der die erste Welle koaxial eingeschlossen ist und ein Ritzel an einem distalen Abschnitt ausgebildet ist; und einem Zahnstangenbasisabschnitt, der mit dem Basisabschnitt verbunden ist, auf dem eine mit dem Ritzel in Eingriff zu bringende Zahnstange in einer annähernd halbkreisförmigen Form ausgebildet ist;
und ein Schnittpunkt der Wellenmittelpunkte der beiden Gelenkwellen des Kardangelenks auf einem axialen Mittelpunkt der Drehachse der Zahnstange positioniert ist.

2. Chirurgisches Instrument nach Anspruch 1, wobei: der erste Umwandlungsmechanismus einen ersten röhrenförmigen Körper umfasst, durch den der erste Schaft hindurchgeht; eine erste schraubenförmige Nut an einer Seitenwand des ersten röhrenförmigen Körpers ausgebildet ist; ein erstes Gleitelement, das durch die erste schraubenförmige Nut zum Gleiten geführt wird, an dem ersten Schaft ausgebildet ist; ein erster röhrenförmiger Körpereingriffsabschnitt, der mit dem ersten Manipulator in Eingriff steht und eine lineare Bewegung vom ersten Manipulator auf den ersten röhrenförmigen Körper überträgt, ist an der Außenfläche des ersten röhrenförmigen Körpers ausgebildet; und wenn der erste Manipulator bewegt wird, wird der erste röhrenförmige Körper linear bewegt, und das erste Gleitelement wird durch die erste schraubenförmige Nut geführt, um die Drehung der ersten Welle zu ermöglichen.

3. Chirurgisches Instrument nach Anspruch 1 oder Anspruch 2, wobei: der zweite Umwandlungsmechanismus einen zweiten röhrenförmigen Körper umfasst, der in Verbindung mit dem Universalgelenk gedreht wird; eine zweite schraubenförmige Nut auf der Seitenfläche des zweiten röhrenförmigen Körpers ausgebildet ist; eine zweite Welle, die mit dem proximalen Ende der Kurbel verbunden ist, vorgesehen ist; ein zweites Gleitelement, das durch die zweite schraubenförmige Nut geführt wird, um zu gleiten, auf der zweiten Welle ausgebildet ist; und wenn der zweite röhrenförmige Körper gedreht wird, das zweite Gleitelement durch die zweite schraubenförmige Nut geführt wird, um zu ermöglichen, dass die zweite Welle eine Gleitbewegung ausführt.

4. Chirurgisches Instrument nach einem der Ansprüche 1 bis 3, versehen mit einer Zangenfunktion zum Greifen eines lebenden Gewebes mittels des beweglichen Teils und des Basisteils des Werkzeugkopfes.

5. Chirurgisches Instrument nach einem der Ansprüche 1 bis 3, das mit Hilfe des beweglichen Teils und des Basisteils des Werkzeugkopfes die Funktion einer Schere zum Schneiden von lebendem Gewebe hat.

6. Chirurgisches Instrument nach einem der Ansprüche 1 bis 3, das mit einer endoskopischen Funktion ausgestattet ist, wobei an dem beweglichen Teil des Werkzeugkopfes eine Kamera angebracht ist, um den Körper von innen zu inspizieren.

## Revendications

1. Instrument chirurgical comprenant :
dans une partie distale, une tête d'outil ayant des degrés de liberté longitudinaux et latéraux ; et dans une partie proximale, un premier manipulateur pour contrôler les degrés de liberté longitudinaux et un second manipulateur pour contrôler les degrés de liberté latéraux ; dans lequel
la tête de l'outil comprend une partie mobile ayant des degrés de liberté longitudinaux et une partie de base qui définit l'orientation latérale de la tête de l'outil,
la partie mobile et le premier manipulateur sont reliés par une première structure de connexion,
la partie de base et le second manipulateur sont reliés par une seconde structure de connexion,
la première structure de connexion comprend : un premier mécanisme de conversion qui convertit le mouvement linéaire du premier manipulateur en mouvement rotatif ; un premier arbre qui doit être mis en rotation par le premier mécanisme de conversion ; un joint universel situé à l'extrémité distale du premier arbre ; un deuxième mécanisme de conversion connecté au joint universel pour convertir le mouvement rotatif en mouvement linéaire ; et une manivelle située à l'extrémité distale du deuxième mécanisme de conversion et connectée à l'extrémité proximale de la partie mobile ;
la seconde structure de liaison est configurée pour transmettre le mouvement de rotation du second manipulateur, et comporte : un arbre tubulaire dans lequel le premier arbre est encastré coaxialement et un pignon est formé dans une partie distale ; et une partie de base de crémaillère reliée à la partie de base, sur laquelle une crémaillère à engrener avec le pignon est formée dans une forme semi-circulaire approximative ;
et une intersection des centres des arbres de deux pivots du joint universel est positionnée sur un centre axial de l'axe de rotation de la crémaillère.

2. L'instrument chirurgical selon la revendication 1, dans lequel : le premier mécanisme de conversion comprend un premier corps tubulaire à travers lequel la première tige pénètre ; une première rainure hélicoïdale est formée sur une paroi latérale du premier corps tubulaire ; un premier élément coulissant qui est guidé par la première rainure hélicoïdale pour glisser est formé sur la première tige ; une partie d'engagement du premier corps tubulaire qui s'engage avec le premier manipulateur et transmet un mouvement linéaire du premier manipulateur au premier corps tubulaire est formée sur la surface extérieure du premier corps tubulaire ; et lorsque le premier manipulateur est déplacé, le premier corps tubulaire est déplacé linéairement, et le premier élément coulissant est guidé par la première rainure hélicoïdale pour permettre au premier arbre de tourner.

3. L'instrument chirurgical selon la revendication 1 ou la revendication 2, dans lequel : le deuxième mécanisme de conversion comprend un deuxième corps tubulaire qui tourne en liaison avec le joint universel ; une deuxième rainure hélicoïdale est formée sur la surface latérale du deuxième corps tubulaire ; un deuxième arbre relié à l'extrémité proximale de la manivelle est prévu ; un deuxième élément de glissement guidé par la deuxième rainure hélicoïdale pour glisser est formé sur le deuxième arbre ; et lorsque le deuxième corps tubulaire est tourné, le deuxième élément de glissement est guidé par la deuxième rainure hélicoïdale pour permettre au deuxième arbre d'effectuer un mouvement de glissière.

4. L'instrument chirurgical selon l'une des revendications 1 à 3, doté d'une fonction de pince pour saisir un tissu vivant, au moyen de la partie mobile et de la partie de base de la tête de l'outil.

5. L'instrument chirurgical selon l'une des revendications 1 à 3, doté d'une fonction de ciseaux pour couper les tissus vivants, au moyen de la partie mobile et de la partie de base de la tête de l'outil.

6. L'instrument chirurgical selon l'une des revendications 1 à 3, doté d'une fonction endoscopique, avec une caméra montée sur la partie mobile de la tête de l'outil pour inspecter l'intérieur du corps.
